# EUROPÄISCHE PATENTSCHRIFT

(11) **EP 0 601 152 B1**
(45) Veröffentlichungstag und Bekanntmachung des Hinweises auf die Patenterteilung: **12.03.1997**
(21) Anmeldenummer: 93912613.2
(22) Anmeldetag: 22.06.1993
(51) Int. Cl.: A61B 17/60

(54) **OSTEOSYNTHESEHILFSMITTEL FÜR DIE FIXATION VON KNOCHEN**
OSTEOSYNTHETIC AUXILIARY FOR THE FIXATION OF BONES
MOYEN AUXILIAIRE D'OSTEOSYNTHESE POUR LA CONTENTION D'OS

(30) Priorität: 26.06.1992 DE 4220936
(43) Veröffentlichungstag der Anmeldung: 15.06.1994
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.
(86) Internationale Anmeldenummer: DE9300546
(87) Internationale Veröffentlichungsnummer: WO9400067

(56) Entgegenhaltungen:
- EP-A- 0 011 258
- EP-A- 0 024 256
- EP-A- 0 420 813
- EP-A- 0 490 812
- EP-A- 0 517 939
- WO-A-88/05287
- WO-A-92/15258
- FR-A- 2 531 332
- FR-A- 2 574 653
- FR-A- 2 628 627
- GB-A- 2 240 043
- US-A- 2 346 346
- US-A- 4 244 360

## Beschreibung

Die Erfindung bezieht sich auf ein Osteosynthesehilfsmittel für die Fixation von Knochen.

Ein gattungsbildendes Osteosynthesehilfsmittel ist aus der WO-A-88052287 bekannt, bei welchem über das Doppelkugelgelenk und das zwischen den beiden Kugelköpfen angeordnete Distanzstück ein großer Verschwenkwinkel der beiden die Fixationsmittel tragenden Elemente gegeneinander möglich wird und bei welchem die Verstellung dieser Elemente für sich in allen Richtungen möglich ist. Durch die schwenkbare und höhenverstellbare Festlegung der Fixationsmittel an den diese tragenden Elementen werden zusätzliche Freiheitsgrade für die Gesamtvorrichtung geschaffen, so daß es möglich ist, auch den kompliziertesten Knochenstellungen und Einrichtarbeiten gerecht zu werden.

Aus der US 42 44 360 ist ein Fixateur bekannt geworden bei dem die Fixationsmittel dadurch festgelegt werden, daß sie in entsprechenden Aufnahmebohrungen verklemmt werden WO-A-9215258, ein Dokument welches unter Art. 54(3) EPÜ fällt, beschreibt einen Fixateur, bei dem die Fixations mittel durch Verklemmen in Aufnahmebohrungen von ineinander verschieblichen Rohren festgelegt sind.

Bei der Anwendung solcher sogenannter äußerlicher Fixateure im Bereich der Hand und der Finger wird zwar einerseits genauso eine große und möglichst weitgehende Verstellung der einzelnen Elemente zueinander angestrebt, andererseits sollen aber die Bauteile so klein sein, daß sie beispielsweise im Bereich der Hand möglichst wenig stören.

Der Erfindung liegt daher die Aufgabe zugrunde, das gattungsbildende Osteosynthesehilfsmittel so auszubilden, daß es möglichst klein ist und dabei mit möglichst wenig Aufwand eine möglichst komplette Arretierung aller einzelnen Bauteile gegeneinander möglich ist.

Diese der Erfindung zugrundeliegende Aufgabe wird durch die Lehre des Hauptanspruches gelöst.

Vorteil hafte Ausgestaltungen sind in den Unteransprüchen erläutert.

Mit anderen Worten ausgedrückt wird vorgeschlagen, daß die eigentlichen Klemmeinrichtungen, die die Fixationsmittel tragen, als flache, beispielsweise rechteckige, quadratische oder ovale Platten ausgebildet sind, wobei die Fixationsmittel in quer zur Plattenebene verlaufende Bohrungen einsetzbar sind. Die Fixationsmittel erstrecken sich also quer zur Plattenebene und die Festlegung der Fixationsmittel in diesen Klemmitteln erfolgt dadurch, daß die Klemmittel wenigstens je aus zwei Platten bestehen, die z. B. über einen Exzenter gegeneinander verschoben werden können, so daß dadurch die Festklemmung der Fixationsmittel erfolgt.

Vorzugsweise besteht jede Klemmeinrichtung aus dem plattenförmigen Bauteil, das in der Plattenebene einen Schlitz aufweist, in den eine Klemmplatte eingesetzt wird, wobei der Exzenter durch die beiden, die Klemmplatte abdeckenden Plattenteile verläuft.

In den plattenförmigen Klemmeinrichtungen sind die Bohrungen zur Aufnahme der Fixationsmittel vorgesehen, wobei vorzugsweise wenigstens einige dieser Bohrungen konvergierend ausgebildet sind, so daß dadurch ein sehr enges Zusammenführen der Enden, beispielsweise von Knochenpins, möglich ist.

Die eigentlichen Klemmeinrichtungen sind auf den sie tragenden Haltestäben frei verschiebbar angeordnet und können durch entsprechende Innensechskantschrauben, die sich in den Klemmeinrichtungen führen, festgelegt werden.

Um auch ein Distrahieren oder Komprimieren der zu verbindenden Knochenteile zu ermöglichen, ist wenigstens einer der Haltestäbe mit einer Widerlagerscheibe ausgerüstet. Der Haltestab weist dabei ein Außengewinde auf und eine entsprechende Bohrung in der Widerlagerscheibe ein Innengewinde, wobei diese beiden Gewinde miteinander kämmen. Je nach Anordnung dieser Widerlagerscheibe auf der einen oder anderen Seite einer Klemmeinrichtung, kann nunmehr diese Klemmeinrichtung nach außen oder innen verschoben werden, und zwar unter entsprechender Kraftaufbringung, so daß dadurch die Distraktion oder Kompression möglich wird, während die andere Klemmeinrichtung über die Fixationsmittel an dem Knochen und über ein Feststellmittel an dem Haltestab festgelegt ist.

Das aus dem Stand der Technik bekannte Doppelkugelgelenk weist auch bei der erfindungsgemäßen Einrichtung ein starres Distanzstück auf, wobei aber dieses Distanzstück als ein Rahmen ausgebildet ist, in dem die beiden Kugeln, die an den Haltestäben angeordnet sind, gehalten werden. Um nunmehr eine Arretierung der eingestellten Haltestäbe zu erreichen, werden über einen Keilschieber innerhalb des Rahmens die beiden Kugeln in ihrer eingestellten Lage festgeklemmt. Auch diese Einrichtung baut relativ klein und dünn, so daß auch diese Einrichtung selbst im Bereich der Hand möglichst wenig stört.

Die Arretierung erfolgt weiterhin vorzugsweise dadurch, daß der Rahmen nicht nur den Keilschieber aufnimmt, sondern noch zwei Klemmstücke, die einerseits eine teilkugelförmige Ausnehmung zur Zusammenarbeit mit den Kugeln der Haltestäbe aufweisen, andererseits mit doppelt geneigten Keilflächen ausgerüstet sind, die einerseits mit dem Keilschieber zusammenwirken, andererseits mit einem durch den Rahmen geführten Betätigungselement, das an seiner Außenseite innerhalb des Raumes des Rahmens konisch sich verjüngend gestaltet ist, so daß durch Betätigen dieses Betätigungselementes nicht nur der Keilschieber angehoben, sondern das Betätigungselement auch etwas nach unten geführt wird und durch diesen doppelten Druck das Verklemmen der Kugeln erfolgt.

Ausführungsbeispiele der Erfindung werden nachfolgend anhand der Zeichnungen erläutert. Die Zeichnungen zeigen dabei in
- Fig. 1: eine schaubildliche Darstellung des Osteosynthesehilfsmittels, in
- Fig. 2: einen Schnitt durch das Osteosynthesehilfsmittel gemäß Fig. 1 in größerem Maßstab, in
- Fig. 3: einen Schnitt gemäß der Linie 3 - 3 in Fig., in
- Fig. 4: eine abgeänderte Formgebung für die Klemmeinrichtungen und in
- Fig. 5: in einer auseinandergezogenen Darstellungsweise den Aufbau des Doppelkugelgelenkes.

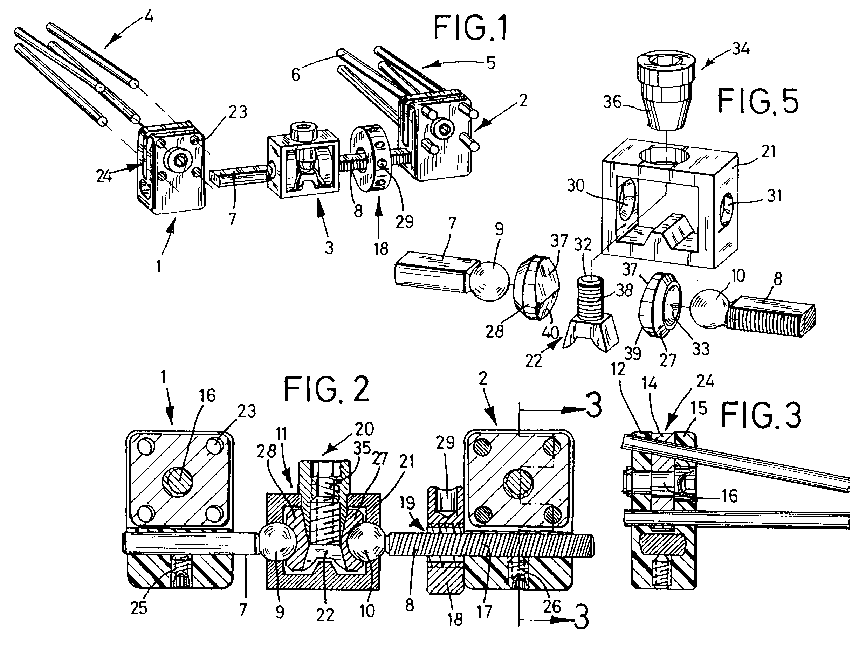

Das eigentliche Osteosynthesehilfsmittel besteht aus den beiden Klemmeinrichtungen 1 und 2, wobei diese Klemmeinrichtungen 1 und 2 Fixationsmittel 4 und 5 tragen, die als Knochenpins oder Schrauben ausgebildet sein können. Bei der Darstellung in Fig. 1 sind Knochenpins 6 dargestellt.

Die beiden Klemmeinrichtungen 1 und 2 werden an Haltestäben 7 und 8 angeordnet, die an ihren aufeinanderzu gerichteten Enden Kugeln 9 und 10 tragen, die so ein Doppelkugelgelenk 3 bilden. Die Kugeln 9 und 10 sind dabei in einem Rahmen 21 gelagert, der somit ein starres Distanzstück bildet. Selbstverständlich sind die Haltestäbe 7 und 8 mit ihren Kugeln 9 und 10 in allen Richtungen verschwenkbar in dem Rahmen 21 gelagert, so daß ein Ausrichten entsprechend der Krümmung der zu schienenden Knochen möglich ist.

Die Haltestäbe 7 und 8 können mit Gewinde ausgerüstet sind. Bi dem dargestellten Ausführungsbeispiel ist der Haltestab 8 mit Außengewinde 17 ausgerüstet ist. Auf dem Haltestab 8 ist eine Widerlagerscheibe 18 vorgesehen, die eine mittlere Bohrung 19 aufweist, die mit Innengewinde ausgerüstet ist, wobei dieses Innengewinde mit dem Außengewinde 17 des Haltestabes 8 kämmt. Hierdurch ist ein Verschieben der auf dem Haltestab 8 angeordneten Klemmeinrichtung 2 möglich, und zwar auch gegen entsprechende Widerstandskräfte. Bei der Anordnung gemäß Fig. 2 wird bei einem Verschieben der Klemmeinrichtung 2 nach rechts eine Distraktion des Knochens erreicht, wenn die Klemmeinrichtung 1 mit ihren Fixationsmitteln 4 an dem feststehenden Knochenteil und über eine Innensechskantschraube 25 an dem Haltestab 7 befestigt ist. Wird die Widerlagerscheibe 18 auf der rechten Seite der Klemmeinrichtung 2 angeordnet, ist eine Kompression der Knochenteile möglich.

Die Festlegung der Fixationsmittel 4 und 5 in Bohrungen 23 in den Klemmeinrichtungen 1 und 2 erfolgt in einfachster Weise dadurch, daß die plattenförmige Klemmeinrichtung 1 und 2 einen mittleren Schlitz 24 aufweist, in dem eine Klemmplatte 14 gelagert ist. Hierdurch werden, wie dies deutlich die Fig. 3 zeigt, drei zusammenwirkende Platten 12, 14 und 15 geschaffen. In den beiden Platten 12 und 15 ist ein Exzenter 16 gelagert, dessen exzentrischer Teil im Bereich der Klemmplatte 14 liegt, so daß durch ein Drehen des Exzenters 16, der ebenfalls als Innensechskant ausgebildet ist, ein Verklemmen der Klemmplatte 14 mit den diese Klemmplatte 14 und die beiden Platten 12 und 15 durchdringenden Fixationsmitteln 4, 5 erfolgt. Hierdurch werden die Fixationsmittel 4, 5 stabil festgelegt.

Die Festlegung der Klemmeinrichtungen 1 und 2 auf den Haltestäben 7 und 8 erfolgt über Innensechskantschrauben 25 und 26.

Die Arretierung der über die Haltestäbe 7 und 8, die Kugeln 9 und 10 und den Rahmen 21 miteinander verbindbaren Klemmeinrichtungen 1 und 2 erfolgt durch eine Arretiervorrichtung 20, indem in dem Rahmen 21 quer zur Längsachse der Haltestäbe 7 und 8 ein Keilschieber 22 verstellbar ist, der bei seiner Verstellung die Kugeln 9 und 10 im Rahmen 21 festlegt. Bei dem in Fig. 2 dargestellten Ausführungsbeispiel sind noch Klemmstücke 27 und 28 erkennbar, die einerseits mit an die Kugelform angepaßten Ausnehmungen und andererseits an die Form des Keilschiebers 22 angepaßten Flächen ausgerüstet sind, so daß bei Verstellen des Keilschiebers 22 eine mittelbare Beaufschlagung der Kugeln 9 und 10 erfolgt.

Die Betätigung der Widerlagerscheibe 18 kann durch Einsetzen entsprechender Stifte in im Außenumfang der Widerlagerscheibe 18 vorgesehene Bohrungen 29 besonders leicht erfolgen.

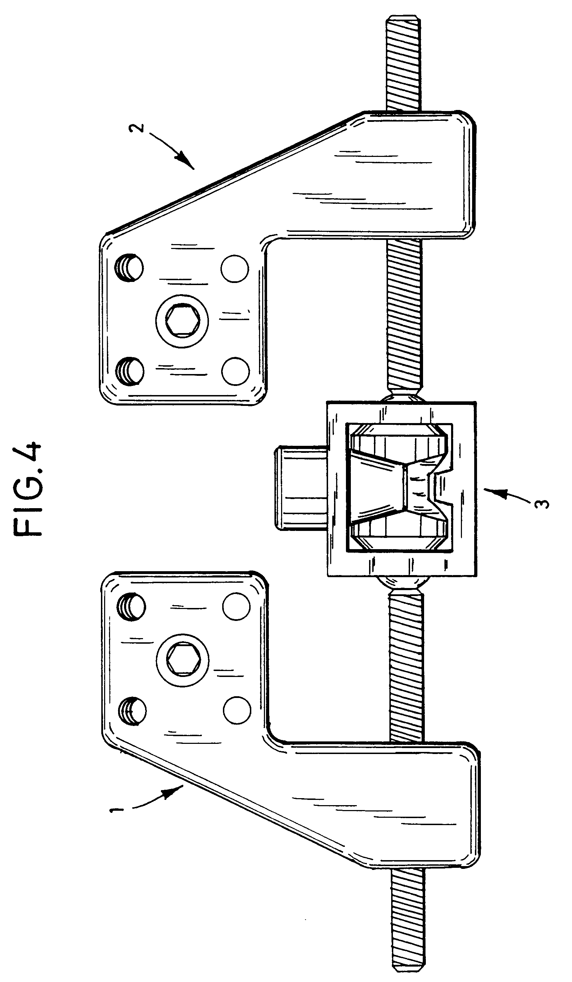

In Fig. 4 sind "L"-förmig ausgebildete Klemmeinrichtungen 1 und 2 dargestellt.

In Fig. 5 ist in einer auseinandergezogenen Darstellungsweise der Aufbau des Doppelkugelgelenkes dargestellt. Es ist ersichtlich, daß die Kugeln 9 und 10 fest an den Haltestäben 7 und 8 angeordnet sind und daß die Arretiervorrichtung 20 im wesentlichen durch einen Rahmen 21 gebildet wird, auf dessen Innenseite teilkugelförmige Ausnehmungen 30 vorgesehen sind, die der Aufnahme der Kugeln 9 bzw. 10 dienen, wie dies in Fig. 2 dargestellt ist. Die Haltestäbe 7 und 8 greifen dabei durch den Rahmen hindurch. Im Inneren des Rahmens ist einerseits ein Keilschieber 22 angeordnet, der einen Gewindezapfen 32 aufweist, der mit Außengewinde 38 versehen ist. Dieser Gewindezapfen 32 kann mit einem Innengewinde 35 eines hülsenförmigen Betätigungselementes 34 zusammenwirken, das an seiner Oberseite mit einer Innenmehrkantöffnung ausgerüstet ist, in die ein entsprechender Betätigungsschlüssel eingesetzt werden kann. Das Betätigungselement 34 durchgreift eine Bohrung im Rahmen 21. Die beiden Kugeln 9 und 10 werden zur Innenseite des Rahmens 21 hin durch Klemmstücke 27 und 28 abgedeckt, die einerseits teilkugelförmige Ausnehmungen 33 für die Kugeln 9 und 10 aufweisen, andererseits mit Doppelkeilflächen ausgebildet sind, wobei die oberen Keilflächen 37 erkennbar sind und die unteren Keilflächen 40 und 39 mit den Keilflächen des Keilschiebers 22 zusammenwirken. Die oberen Keilflächen 37 arbeiten mit einer konischen Fläche 36 des Betätigungselementes 34 zusammen, so daß bei einem Aufeinanderzubewegen der beiden Bauteile 22 und 34 ein Nachaußendrücken der Klemmstücke 27 und 28 erfolgt, durch die die Kugeln 9 und 10 arretiert werden, während umgekehrt bei einem Auseinanderbewegen des Betätigungselementes 34 und des Keilschiebers 22 die Klemmstücke 27 und 28 gegenüber den Kugeln 9 und 10 gelockert werden, so daß damit eine Verstellung der Haltestäbe 7 und 8 erfolgen kann.

## Patentansprüche

1. Osteosynthesehilfsmittel für die Fixation von Knochen mit zwei jeweils Fixationsmittel (4, 5) tragenden Klemmeinrichtungen (1, 2), die über ein arretierbares Doppelkugelgelenk (3) und Haltestäbe (7, 8) miteinander verbindbar sind, wobei die Fixationsmittel (4, 5), wie Schrauben- oder Knochenpins (6) an jeder Klemmeinrichtung (1, 2) arretierbar verstellbar sind und die Kugeln (9, 10) des Doppelkugelgelenkes (3) über ein starres Distanzstück (11) miteinander verbunden sind, wobei die Klemmeinrichtungen (1, 2) als flache Platten ausgebildet sind, die quer zur Plattenebene verlaufende Bohrungen (23) zur Aufnahme der Fixationsmittel (4, 5) aufweisen und jede Klemmeinrichtung (1, 2) aus wenigstens zwei Platten (12, 14, 15) besteht, die mittels Betätigungsmittel so gegeneinander verschiebbar sind, daß ein Verklemmen der Platten mit den diese Platt durchdringenden Fixationsmitteln (4, 5) erfolgt.

2. Osteosynthesehilfsmittel nach Anspruch 1, wobei die Betätigungsmittel als Excenter (16) ausgebildet sind.

3. Osteosynthesehilfsmittel nach Anspruch 1 oder 2, wobei die Achsen wenigstens einiger Bohrungen (23) in jeder Klemmeinrichtung (1, 2) konvergieren.

4. Osteosynthesehilfsmittel nach einem der vorhergehenden Ansprüche, wobei die Klemmeinrichtungen (1, 2) auf den Haltestäben (7, 8) frei verstellbar und arretierbar sind.

5. Osteosynthesehilfsmittel nach einem der vorhergehenden Ansprüche, wobei wenigstens ein Haltestab (7, 8) mit Außengewinde (17) versehen ist und eine Widerlagerscheibe (18) trägt, die eine Bohrung (19) aufweist, die mit Innengewinde ausgerüstet ist, das mit dem Außengewinde (17) des Haltestabes (7, 8) kämmt.

6. Osteosynthesehilfsmittel nach einem der vorhergehenden Ansprüche, wobei das starre Distanzstück (11) mit einer Arretiervorrichtung (20) zur Arretierung der beiden Kugeln (9, 10) ausgerüstet ist.

7. Osteosynthesehilfsmittel nach Anspruch 6, wobei das starre Distanzstück (11) aus einem Rahmen (21) besteht in dem die Kugeln (9, 10) gehalten und durch einen im Rahmen (21) geführten Keilschieber (22) im Rahmen (21) festklemmbar sind.

8. Osteosynthesehilfsmittel nach einem der vorhergehenden Ansprüche, wobei jede plattenförmige Klemmeinrichtung einen in der Plattenebene ausgerichteten Schlitz (24) aufweist, in dem eine Klemmplatte (14) angeordnet ist, die durch den Exzenter (16) verstellbar ist.

9. Osteosynthesehilfsmittel nach einem der vorhergehenden Ansprüche,
wobei der
Rahmen (21) des Doppelkugelgelenkes (3) an seiner Innenseite zwei teilkugelförmige Ausnehmungen (30) als Sitz zur Aufnahme der Kugeln (9, 10) besitzt,
die Kugeln (9, 10) die Haltestäbe (7, 8) tragen,
der Rahmen (21) Öffnungen (31) im Bereich der Ausnehmungen (30) aufweist, durch die die Haltestäbe (7, 8) nach außen geführt sind, und
der Keilschieber (22) zwischen den auf ihren Sitzen angeordneten Kugeln (9, 10) verstellbar angeordnet ist, so daß durch Verstellen des Keilschiebers (22) die Kugeln (9, 10) auf ihren Sitzen festklemmbar sind.

10. Osteosynthesehilfsmittel nach Anspruch 9, wobei der Keilschieber (22) mit einem Gewindezapfen (32) ausgerüstet ist, der den Rahmen (21) durchquert, wodurch der Keilschieber (22) gegenüber dem Rahmen (21) verstellbar und an dem Rahmen (21) arretierbar ist.

11. Osteosynthesehilfsmittel nach einem der vorhergehenden Ansprüche, wobei zwischen dem Keilschieber (22) und jeder Kugel (9, 10) je ein Klemmstück (27, 28) angeordnet ist, das jeweils einerseits eine mit dem Keilschieber (22) zusammenwirkende Keilfläche (39, 40) und andererseits eine mit der Kugel (9, 10) zusammenwirkende teilkugelförmige Ausnehmung (33) aufweist.

12. Osteosynthesehilfsmittel nach einem der vorhergehenden Ansprüche, wobei der Gewindezapfen (32) mit einem den Rahmen (11) durchquerenden hülsenförmigen Betätigungselement (34) zusammenwirkt, das mit einem Innengewinde (35) ausgerüstet ist, das mit dem Außengewinde (38) des Gewindezapfens (32) zusammenwirkt und das an seiner Außenseite innerhalb des Raums des Rahmens (21) konisch sich verjüngend (bei 36) gestaltet ist und mit entsprechenden kegelförmigen Gegenflächen (37) der Klemmstücke (27 und 28) zusammenwirkt, um somit die Klemmstücke (27, 28) mit den Kugeln (9, 10) zu verklemmen oder die Kugeln gegenüber den Klemmstücken (27, 28) zu lösen.

13. Osteosynthehilfsmittel nach einem der vorhergehenden Ansprüche, wobei die Klemmstücke (27, 28) je mit Doppelkeilflächen (40, 37; 39, 37) ausgerüstet sind, von denen die eine (37) den Konusflächen (36) des Betätigungselementes (34) und die anderen (40, 39) den Keilflächen des Keilschiebers (22) anliegen.

## Claims

1. An osteosynthetic aid for fixing bones, comprising two clamping devices (1, 2) each carrying fixing means (4, 5), which clamping devices (1, 2) may be connected to each other by means of a lockable double ball-and-socket joint (3) and holding bars (7, 8), the fixing means (4, 5), such as screw or bone pins (6), on each clamping device (1, 2) being arrestably adjustable and the balls (9, 10) of the double ball-and-socket joint (3) being connected together by means of a rigid spacer member (11), wherein the clamping devices (1, 2) are constructed as flat plates which comprise holes (23) extending perpendicularly to the plate plane to accommodate the fixing means (4, 5), and each clamping device (1, 2) consists of at least two plates (12, 14, 15) displaceable with respect to each other by means of actuating means in such a way that clamping of the plates with the fixing means (4, 5) penetrating these plates is effected.

2. An osteosynthetic aid according to claim 1, wherein the actuating means are constructed as eccentrics (16).

3. An osteosynthetic aid according to claim 1 or claim 2, wherein the axes of at least some of the holes (23) in each clamping device (1, 2) converge.

4. An osteosynthetic aid according to any one of the preceding claims, wherein the clamping devices (1, 2) may be freely adjusted and locked on the holding bars (7, 8).

5. An osteosynthetic aid according to any one of the preceding claims, wherein at least one holding bar (7, 8) is provided with an external thread (17) and carries an abutment disk (18) comprising a hole (19) provided with an internal thread which meshes with the external thread (17) on the holding bar (7, 8).

6. An osteosynthetic aid according to any one of the preceding claims, wherein the rigid spacer member (11) is provided with a locking device (20) for locking the two balls (9, 10).

7. An osteosynthetic aid according to claim 6, wherein the rigid spacer member (11) consists of a frame (21) in which the balls (9, 10) are held and may be clamped firmly within the frame (21) by a wedge-shaped sliding member (22) guided in the frame (21).

8. An osteosynthetic aid according to any one of the preceding claims, wherein each plate-shaped clamping device comprises a slit (24) oriented in the plate plane, in which slit (24) there is arranged a clamping plate (14) adjustable by the eccentric (16).

9. An osteosynthetic aid according to any one of the preceding claims, wherein the frame (21) of the double ball-and-socket joint (3) comprises two recesses (30) in the form of part of a sphere on the inside as seats for accommodating the balls (9, 10), the balls (9, 10) carry the holding bars (7, 8), the frame (21) comprises openings (31) in the area of the recesses (30), through which openings (31) the holding bars (7, 8) are guided outwards, and the wedge-shaped sliding member (22) is arranged adjustably between the balls (9, 10) arranged on their seats, such that the balls (9, 10) may be clamped to their seats by adjustment of the wedge-shaped sliding member (22).

10. An osteosynthetic aid according to claim 9, wherein the wedge-shaped sliding member (22) is provided with a threaded pin (32) which passes through the frame (21), whereby the wedge-shaped sliding member (22) may be adjusted with respect to the frame (21) and locked to the frame (21).

11. An osteosynthetic aid according to any one of the preceding claims, wherein clamping members (27, 28) are arranged between the wedge-shaped sliding member (22) and each ball (9, 10), each of which clamping members (27, 28) comprises on the one hand a wedge face (39, 40) interacting with the wedge-shaped sliding member (22) and on the other hand comprises a recess (33) in the form of part of a sphere interacting with the ball (9, 10).

12. An osteosynthetic aid according to any one of the preceding claims, wherein the threaded pin (32) interacts with a sleeve-shaped actuating element (34) passing through the frame (11) and provided with an internal thread (35) which interacts with the external thread (38) on the threaded pin (32), which actuating element (34) tapers conically (at 36) on its outside inside the frame (21) and interacts with corresponding cone-shaped counterfaces (37) on the clamping members (27 and 28), in order to clamp the clamping members (27, 28) with the balls (9, 10) or to release the balls with respect to the clamping members (27, 28).

13. An osteosynthetic aid according to any one of the preceding claims, wherein the clamping members (27, 28) are each provided with double wedge faces (40, 37; 39, 37), one (37) of which adjoins the cone faces (36) of the actuating element (34) and the others (40, 39) of which adjoin the wedge faces of the wedge-shaped sliding member (22).

## Revendications

1. Moyen auxiliaire d'ostéosynthèse pour la contention d'os comprenant deux dispositifs de serrage (1, 2), sur lesquels sont fixées les pièces contentives (4, 5) et qui peuvent être reliés l'un à l'autre par une double articulation sphérique (3), qu'il est possible de bloquer, et des tiges de support (7, 8), les pièces contentives (4, 5), telles que des vis ou des broches (6), pouvant être bloquées sur chaque dispositif de serrage (1, 2) et les organes sphériques (9, 10) de la double articulation sphérique (3) étant reliés l'un à l'autre par une pièce d'écartement rigide (11), dans lequel les dispositifs de serrage (1, 2) sont conçus en forme de plaques planes, qui sont munies de forures (23), réalisées transversalement par rapport au plan de la plaque, dans lesquelles s'engagent les pièces contentives (4, 5), et chaque dispositif de serrage (1, 2) est formé de deux plaques au moins (12, 14, 15), qui peuvent se déplacer l'une contre l'autre à l'aide d'organes de manoeuvre, de telle sorte que les plaques sont bloquées avec les pièces contentives (4, 5), qui sont engagées dans ces plaques.

2. Moyen auxiliaire d'ostéosynthèse selon la revendication 1, dans lequel les organes de manoeuvre sont conçus sous forme d'excentriques (16).

3. Moyen auxiliaire d'ostéosynthèse selon la revendication 1 ou 2, dans lequel au moins quelques forures (23), réalisées dans chaque dispositif de serrage (1, 2), convergent.

4. Moyen auxiliaire d'ostéosynthèse selon l'une quelconque des revendications précédentes, dans lequel les dispositifs de serrage (1, 2) peuvent se déplacer librement sur les tiges de support (7, 8), sur lesquelles il est possible de les bloquer.

5. Moyen auxiliaire d'ostéosynthèse selon l'une quelconque des revendications précédentes, dans lequel au moins une tige de support (7, 8) est munie d'un filetage extérieur (17) et porte un disque de butée (18), qui est muni d'une forure (19), laquelle est munie d'un filetage intérieur qui s'engage dans le filetage extérieur (17) de la tige de support (7, 8).

6. Moyen auxiliaire d'ostéosynthèse selon l'une quelconque des revendications précédentes, dans lequel la pièce d'écartement rigide (11) est munie d'un dispositif de blocage (20), destiné à bloquer les deux organes sphériques (9, 10).

7. Moyen auxiliaire d'ostéosynthèse selon la revendication 6, dans lequel la pièce d'écartement rigide (11) est formée par un cadre (21), dans lequel sont maintenus les organes sphériques (9, 10), lesquels peuvent être bloqués dans le cadre (21) par un coulisseau conique (22), qui se déplace dans le cadre (21).

8. Moyen auxiliaire d'ostéosynthèse selon l'une quelconque des revendications précédentes, dans lequel chaque dispositif de serrage en forme de plaque est muni d'une fente (24), orientée dans le plan de la plaque, dans laquelle on introduit une plaque de serrage (14), qui peut être déplacée à l'aide de l'excentrique (16).

9. Moyen auxiliaire d'ostéosynthèse selon l'une quelconque des revendications précédentes, dans lequel
les faces intérieures du cadre (21) de la double articulation sphérique (3) sont munies chacune d'un évidement (30), formant une partie de sphère, destiné à recevoir les organes sphériques (9, 10),
les organes sphériques (9, 10) portent les tiges de support (7, 8),
le cadre (21) est muni d'orifices (31), réalisés dans les évidements (30), par lesquels s'engagent vers l'extérieur les tiges de support (7, 8), et
le coulisseau conique (22), monté entre les organes sphériques (9, 10) engagés dans leur logement respectif, est disposé de manière à pouvoir se déplacer, de telle sorte qu'un déplacement du coulisseau conique (22) permet de bloquer les organes sphériques (9, 10) dans leurs logements.

10. Moyen auxiliaire d'ostéosynthèse selon la revendication 9, dans lequel le coulisseau conique (22) est muni d'un tenon fileté (32), qui traverse une paroi du cadre (21), ce qui permet de déplacer le coulisseau conique (22) par rapport au cadre (21) et de le bloquer contre le cadre (21).

11. Moyen auxiliaire d'ostéosynthèse selon l'une quelconque des revendications précédentes, dans lequel une pièce de serrage (27, 28) est montée entre le coulisseau conique (22) et chaque organe sphérique (9, 10), l'une des faces de ladite pièce de serrage est formée d'une surface oblique (39, 40) agissant conjointement avec le coulisseau conique (22), et l'autre face est munie d'un évidement (33) formant une partie de sphère pour agir conjointement avec l'organe sphérique (9, 10).

12. Moyen auxiliaire d'ostéosynthèse selon l'une quelconque des revendications précédentes, dans lequel le tenon fileté (32) agit conjointement avec un élément de commande (34) en forme de douille, qui traverse le cadre (11) et qui est muni d'un filetage intérieur (35) pour agir conjointement avec le filetage extérieur (38) du tenon fileté (32) et dont la surface extérieure, à l'intérieur de l'espace du cadre (21), se rétrécit (en 36) pour former un cône dont les contre-surfaces (37) coniques agissent conjointement avec les pièces de serrage (27 et 28), ce qui permet de bloquer les pièces de serrage (27, 28) avec les organes sphériques (9, 10) ou de desserrer les organes sphériques (9, 10) par rapport aux pièces de serrage (27, 28).

13. Moyen auxiliaire d'ostéosynthèse selon l'une quelconque des revendications précédentes, dans lequel les pièces de serrage (27, 28) sont formées d'une surface à deux plans (40, 37 ; 39, 37), dont l'un des plans (37) s'appuie contre les surfaces coniques (36) de l'élément de commande (34) et l'autre plan (40, 39) s'appuie contre les surfaces coniques du coulisseau conique (22).
